# EUROPÄISCHE PATENTSCHRIFT

(11) **EP 0 802 193 B1**
(45) Veröffentlichungstag und Bekanntmachung des Hinweises auf die Patenterteilung: **17.10.2001**
(21) Anmeldenummer: 97105137.0
(22) Anmeldetag: 26.03.1997
(51) Int. Cl.: C07D 239/34, C07D 239/46, C07D 239/52, C07D 239/56, C07D 239/60, C07D 405/12, C07D 401/12

(54) **Verfahren zur Herstellung von substituierten Pyrimidinen**
Process for the preparation of substituted pyrimidines
Procédé pour la préparation de pyridines substituées

(30) Priorität: 09.04.1996 CH 89396
(43) Veröffentlichungstag der Anmeldung: 22.10.1997
(73) Patentinhaber: Lonza AG, 3930 Visp (CH)
(72) Erfinder: Bessard, Ives, Dr., 3960 Sierre (Kanton Wallis) (CH); Stucky, Gerhard, Dr., 3902 Brig-Glis (Kanton Wallis) (CH)
(74) Vertreter: Winter, Brandl, Fürniss, Hübner, Röss, Kaiser, Polte Partnerschaft

(56) Entgegenhaltungen:
- EP-A- 0 562 510
- DATABASE WPI Section Ch, Week 9141 Derwent Publications Ltd., London, GB; Class C02, AN 91-300282 XP002035922 & JP 03 200 784 A (ISHIHARA SANGYO KAISHA LTD) , 2.September 1991
- JOURNAL OF MEDICINAL CHEMISTRY, Bd. 27, Nr. 7, 1.Juli 1984, Seiten 1621-1629, XP000196418 D'ATRI G ET AL: "NOVEL PYRIMIDINE AND 1,3,5-TRIAZINE HYPOLIPIDEMIC AGENTS"
- CHEMICAL ABSTRACTS, vol. 46, no. 10, 25.Mai 1952 Columbus, Ohio, US; OHTA ET AL.: "Syntheses of pyrimidyl sulfone derivatives" Seite 4549; XP002035920
- CHEMICAL ABSTRACTS, vol. 44, no. 3, 10.Februar 1950 Columbus, Ohio, US; BUDESINSKY ET AL.: "Preparation of asymmetric sulfones" Seite 1050; XP002035921

## Beschreibung

Die Erfindung betrifft ein neues Verfahren zur Herstellung von substituierten Pyrimidinderivaten der allgemeinen Formel 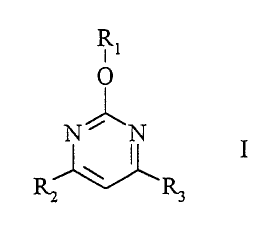 worin R₁, R₂ und R₃ die nachstehende Bedeutung haben.

Diese Verbindungen der allgemeinen Formel I sind wichtige Vorstufen für die Herstellung von z.B. herbizidwirksamen Wirkstoffen (vgl. EP-A-0562 510). Die Verbindungen der allgemeinen Formel I können ein asymmetrisches C-Atom in der Nachbarstellung der Carbonylgruppe im Substituenten R₁ besitzen. Die nachfolgenden Ausführungen umfassen deshalb sowohl die enantiomerenreinen Verbindungen, als auch die Racemate bzw. beliebige Mischungen der Enantiomere untereinander.

Verfahren zur Herstellung der substituierten Pyrimidine der allgemeinen Formel I sind bekannt. So beschreibt die EP-A 562 510 die Herstellung des L-2-(4,6-Dimethoxypyrimidin-2-yloxy)-3-methylbutansäuremethylesters durch Umsetzung von 2-Benzylsulfonyl-4,6-dimethoxypyrimidin mit der L-α-Hydroxyisovaleriansäure in Gegenwart von Kaliumcarbonat. Ähnliche Umsetzungen mit Sulfonylverbindungen werden von G. d'Atri et al. (J. Med. Chem., 1984, 27:1621-1629) und in der JP-A-3200784 beschrieben. Problematisch und aufwendig ist jedoch die Bereitstellung der Sulfonylverbindung, welche in der Regel ausgehend von 2-Thio-4,6-dihydroxypyrimidin über mehrere Verfahrensschritte hergestellt werden muss. Ein Verfahren zur Herstellung von Sulfonen durch Umsetzung von Natrium-p-toluolsulfinat mit 2-Chlor-4-amino-6-methylpyrimidin bzw. 2-Chlor-4-aminopyrimidin wird außerdem von M. Ohta und R. Sudo (J. Pharm. Soc. Japan 71, 514-15 (1951), zitiert in CA 46(10):4549h) beschrieben.

In der Folge wurde versucht das 2-Chlor-4,6-dimethoxypyrimidin direkt mit einer Hydroxyverbindung umzusetzen. Diese Umsetzung verlief jedoch so langsam und unvollständig, dass sich die Suche nach einem neuen Weg aufdrängte.

Die Aufgabe der Erfindung bestand folglich darin, einen einfachen und im technischen Massstab durchführbaren Zugang zu den Verbindungen der allgemeinen Formel I zu finden.

Die Aufgabe konnte gelöst werden mit dem erfindungsgemässen Verfahren gemäss Anspruch 1.

In den folgenden allgemeinen Formeln I bis IV haben die angegebenen Reste folgende Bedeutung:

Alkyl, entweder alleine oder als Bestandteil von Alkoxy, Alkylcarbonyl, Alkoxycarbonyl, Alkylthio, Alkylamino, Dialkylamino, Haloalkoxy, Arylalkyl oder dergleichen kann jeweils geradkettig oder verzweigt sein und umfaßt 1 bis 6 C-Atome, bevorzugt 1 bis 4 C-Atome. Wörtlich genannt seien Methyl, Ethyl, n- oder i-Propyl, n-, i-, t-Butyl, Pentyl und seine Isomeren oder Hexyl und seine Isomeren.
Unter Aryl wird eine gegebenenfalls substituierte Phenyl oder Naphthylgruppe, bevorzugt eine Phenylgruppe verstanden. Arylalkyl steht folglich zweckmässig für Phenyl-(C₁-C₄)-alkyl, insbesondere für Benzyl, und Aryloxy bevorzugt für Phenoxy.
Sowohl Alkyl als auch Aryl kann einen oder mehrere Substituenten, zweckmässig aus der Reihe (C₁-C₄)-Alkyl; Halogen, z.B. Fluor oder Chlor; (C₁-C₄)-Haloalkyl wie z.B. Trifluormethyl; (C₁-C₄)-Alkoxy wie z.B. Methoxy oder Ethoxy; (C₁-C₄)-Haloalkoxy, Nitro oder Cyano, aufweisen. Geeignete Halogene sind Fluor, Chlor und Brom oder Jod, insbesondere Fluor oder Chlor. Entsprechend kann Haloalkoxy z.B. für Trifluormethoxy oder Trichlormethoxy stehen.

Erfindungsgemäss erfolgt die Herstellung der substituierten Pyrimidinderivate der allgemeinen Formel 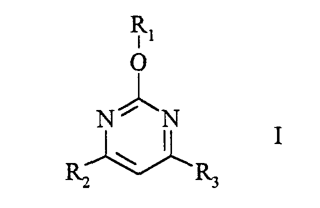 worin R₁ die Bedeutung hat von
i) 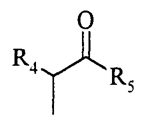 worin R₄ die Bedeutung hat von H, Alkyl, Aryl, Arylalkyl, Carboxyl, Alkylcarbonyl oder von Alkoxycarbonyl und worin R₅ die Bedeutung hat von Hydroxy, Alkoxy, Aryloxy oder Alkyl;
ii) 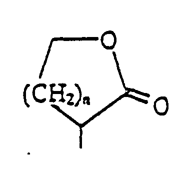 worin n die Bedeutung hat von 1 oder 2, und
iii) 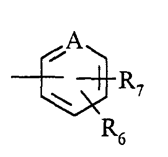 worin A die Bedeutung hat von CH oder von N und worin R₆ und R₇ gleich oder verschieden sind und Wasserstoff, Alkyl, Alkoxy, Aryl, Aryloxy oder eine Gruppe

   -COOR₈

   bedeuten, worin R₈ die Bedeutung hat von Wasserstoff, Alkyl oder Aryl;
R₂ und R₃ gleich oder verschieden sind und H, Alkyl, Alkoxy, Alkylthio, Halogen, Haloalkoxy, Amino, Alkylamino, Dialkylamino bedeuten, durch Umsetzung von einem Halogenpyrimidin der allg. Formel 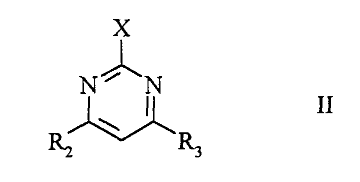 worin X ein Halogenatom bedeutet und R₂ und R₃ die genannte Bedeutung haben, mit einer Hydroxyverbindung ausgewählt aus der Reihe 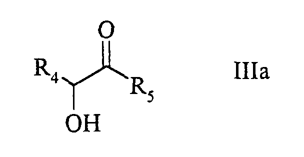 worin R₄ und R₅ die genannte Bedeutung haben; 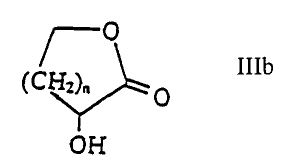 worin n die genannte Bedeutung hat; und 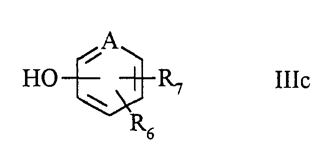 worin A, R₆ und R₇ die genannte Bedeutung haben,
in Gegenwart von einem Sulfinat der allgemeinen Formel

R₉SO₂⁻M⁺ IV

worin R₉ H, Alkyl, Aryl, Arylalkyl oder Alkoxyaryl und M ein Alkali- oder Erdalkalimetallatom bedeuten und in Gegenwart einer anorganischen oder organischen Base zum Endprodukt.

Das Halogenpyrimidin der allgemeinen Formel II als Ausgangsprodukt des erfindungsgemässen Verfahrens kann auf einfache Weise und im technischen Massstab gemäss dem Verfahren z.B. der EP-A 0 582 288 bereit gestellt werden.

Die Hydroxyverbindungen ausgewählt aus der Reihe der allgemeinen Formeln IIIa, IIIb, IIIc sind in der Regel kommerziell erhältlich oder können nach gängigen Methoden hergestellt werden.

Die Hydroxyverbindungen werden zweckmässig in stöchiometrischen Mengen bezogen auf das Halogenpyrimidin der allgemeinen Formel II eingesetzt.

Die erfindungsgemässe Umsetzung wird als sogenannte "Eintopfsynthese" durchgeführt.

Gemäss der "Eintopfsynthese" wird das Sulfinat der allgemeinen Formel IV in katalytischen Mengen von zweckmässig 1 bis 25 Mol%, vorzugsweise 5 Mol% bis 10 Mol%, bezogen auf das eingesetzte Halogenpyrimidin der allgemeinen Formel II, eingesetzt.
Als Sulfinate der allgemeinen Formel IV werden zweckmässig Verbindungen eingesetzt, worin R₉ die Bedeutung hat von (C₁-C₄)-Alkyl, Phenyl-(C₁-C₄)-alkyl oder gegebenenfalls mit (C₁-C₄)-alkyl oder (C₁-C₄)-alkoxy-substituiertes Phenyl. Bevorzugt hat R₉ die Bedeutung von Methyl, p-Methylphenyl, p-Methoxyphenyl oder Benzyl. M⁺ hat zweckmässig die Bedeutung von einem Alkalimetallatom, ausgewählt aus der Reihe Natrium, Kalium und Lithium, bevorzugt von Natrium. Bevorzugte Sulfinate sind infolgedessen das Natriummethansulfinat, das Natrium-p-toluolsulfinat oder das Natrium-benzyl-sulfinat.
Die erfindungsgemässe Umsetzung erfolgt in Gegenwart einer anorganischen oder organischen Base. Geeignete Vertreter von organischen Basen sind tertiäre Amine wie z.B.Triethylamin oder das als Hünigs Base bekannte Diisopropylethylamin..
Bevorzugt hingegen ist die Anwendung von anorganischen Basen wie z.B. die Alkali- oder Erdalkalicarbonate, insbesondere Alkalicarbonate wie z.B. das Kaliumcarbonat oder das Natriumcarbonat. Zweckmässig wird die Base in einer Menge von 1 Moläquivalenten bis 3 Moläquivalenten, vorzugsweise von 1 Moläquivalenten bis 1,5 Moläquivalenten, bezogen auf das eingesetzte Halogenpyrimidin der allgemeinen Formel II, eingesetzt.

Von Vorteil erfolgt die Reaktion in Gegenwart eines gegenüber den Reaktionsteilnehmern inerten und polaren Lösungsmittels. Als besonders geeignete Lösungsmittel haben sich N,N-Dimethylformamid, Sulfolan, Dioxan, Dimethylsulfoxid oder Glykolether wie z.B. das Diglyme, insbesondere aber N,N-Dimethylformamid, herausgestellt.

Die Reaktion erfolgt zweckmässig bei einer Temperatur zwischen 50 °C und 150 °C, bevorzugt zwischen 90 °C und 110 °C, von Vorteil unter weitgehendem Ausschluss von Wasser.

Nach einer Umsetzungszeit von ungefähr 0,5 h bis 24 h kann das Endprodukt der allgemeinen Formel I in guten Ausbeuten bis zu 90 % auf fachmännische Art und Weise, z.B. durch Extraktion aus dem Reaktionsgemisch, isoliert werden.

### Beispiele

### Beispiel 1a (Eintopfverfahren)

### Herstellung von (+/-)-2-(4,6-Dimethoxypyrimidin-2-yl-oxy)-3,3-dimethylbutansäuremethylester

4,38 g(25 mmol) 2-Chlor-4,6-dimethoxypyrimidin, 3,90 g (25 mmol) (+/-)-2-Hydroxy-3,3-dimethylbutansäuremethylester und 0,66 g (6,3 mmol) Natriummethansulfinat wurden in Gegenwart von 5,17 g (37,5 mmol) Kaliumcarbonat in 25 ml N,N-Dimethylformamid unter Rühren auf 120 °C erhitzt. Nach 2 h wurde das Lösungsmittel bei 70 °C / 20 mbar am Rotavapor entfernt. Der Rückstand wurde aufgenommen in 30 ml Wasser und 30 ml Dichlormethan.
Nach Abtrennen der organischen Phase wurde die wässrige Phase erneut mit 20 ml Dichlormethan extrahiert. Die vereinigten organischen Phasen wurden mit Wasser gewaschen, getrocknet über Magnesiumsulfat und eingedampft. Das Titelprodukt wurde in einer Ausbeute von 5,93 g (82,7% d. Theorie) in Form von leicht gelblichen Kristallen erhalten (Gehalt nach GC 99,2%).
Fp. 104,4 °C - 107,0 °C.
   - ¹H NMR (DMSO, MHz 400) δ: 5,88 (1H, s);
   4,70 (1 H, s);
   3,85 (6H, s);
   3,65 (3H, s); 1,07 (9 H, s).
MS: 284 (MP), 269, 228, 196, 169, 157.

### Beispiel 1b (Eintopfverfahren)

### Herstellung von S-(+)-(4,6-Dimethoxypyrimidin-2-yl-oxy)-3,3-dimethylbutansäuremethylester

1,75 g(10 mmol) 2-Chlor-4,6-dimethoxypyrimidin, 1,49 g (10 mmol) S-(+)-2-Hydroxy-3,3-dimethylbutansäuremethylester und 0,45 g (2,5 mmol) Natrium-p-toluolsulfinat wurden in Gegenwart von 2,07 g (15 mmol) Kaliumcarbonat in 10 ml N,N-Dimethylformamid unter Rühren auf 120 °C erhitzt. Nach 7 h wurde das Lösungsmittel bei 60 °C / 20 mbar am Rotavapor entfernt. Der Rückstand wurde aufgenommen in 30 ml Wasser und 30 ml Dichlormethan.
Nach Abtrennen der organischen Phase wurde die wässrige Phase erneut mit 20 ml Dichlormethan extrahiert. Die vereinigten organischen Phasen wurden mit Wasser gewaschen, getrocknet über Magnesiumsulfat und eingedampft. Der Rückstand wurde über eine Silicagel-Säule (Eluationsmittel Hexan/ Essigsäureethylester 4:1) chromatographisch gereinigt. Aus der Produktfraktion wurde das Titelprodukt in Form von leicht gelblichen Kristallen in einer Ausbeute von 1,6 g (56,3% d. Theorie) erhalten (Gehalt nach GC 99%).
Fp. 111,4°C- 114,8°C.
Enantiomerenverhältnis S/R=96,7 / 3,3
   - ¹H NMR (DMSO, MHz 400) δ: 5,88 (1H, s);
   4,70 (1 H, s);
   3,82 (6H, s);
   3,65 (3H, s);
   1,04 (9 H, s).

### Beispiel 1c (Eintopfverfahren) Vergleich ohne Sulfinatzugabe

### Herstellung von (+/-)-2-(4,6-Dimethoxypyrimidin-2-yl-oxy)-3,3-dimethylbutansäuremethylester

Die Umsetzung wurde entsprechend Beispiel 1a, aber ohne Zugabe von Natriumsulfinat durchgeführt.
Nach einer Reaktionszeit von 24 h war ein Eduktumsatz von 54% erreicht.

### Beispiel 2 (Eintopfverfahren)

### Herstellung von (+/-)-3-(4,6-Dimethoxypyrimidin-2-yl-oxy)-butan-2-on

4,38 g (25 mmol) 2-Chlor-4,6-dimethoxypyrimidin, 2,31 g (26,2 mmol) 3-Hydroxy-2-oxobutan und 0,66 g (6,3 mmol) Natriummethansulfinat wurden in Gegenwart von 5,17 g (37,5 mmol) Kaliumcarbonat in 25 ml N,N-Dimethylformamid unter Rühren auf 120 °C erhitzt. Nach 3 h wurde das Lösungsmittel bei 70 °C / 20 mbar am Rotavapor entfernt. Der Rückstand wurde über eine Silicagel-Säule (Eluationsmittel Hexan/ Essigsäureethylester 4:1) chromatographisch gereinigt. Aus der Produktfraktion wurde das Titelprodukt in Form eines leicht gelblichen Öls in einer Ausbeute von 4,58 g (80,4% d. Theorie) erhalten (Gehalt nach GC 99%).
- ¹H NMR (DMSO, MHz 400) δ: 5,87(1 H,s);
5,70 (1 H, q);
3,82 (6 H, s);
2,25 (3 H, s);
1,44 (3 H, d).
MS:**226**;211; 183; 157; 139

### Beispiel 3 (Eintopfverfahren)

### Herstellung von (+/-)-3-(4,6-Dimethoxypyrimidin-2-yl-oxy)-dihydrofuran-2-on

1,75 g (10 mmol) 2-Chlor-4,6-dimethoxypyrimidin, 1,02 g (10 mmol) α-Hydroxy-γ-butyrolacton und 0,26 g (2,5 mmol) Natriummethansulfinat wurden in Gegenwart von 2,07 g (15,0 mmol) Kaliumcarbonat in 10 ml N,N-Dimethylformamid unter Rühren auf 120 °C erhitzt. Nach 2 h wurde das Lösungsmittel bei 70 °C / 20 mbar am Rotavapor entfernt. Der Rückstand wurde aufgenommen in 30 ml Wasser und 30 ml Dichlormethan. Nach Abtrennen der organischen Phase wurde die wässrige Phase erneut mit 20 ml Dichlormethan extrahiert. Die vereinigten organischen Phasen wurden mit Wasser gewaschen, getrocknet über Magnesiumsulfat und eingedampft. Das Titelprodukt wurde in einer Ausbeute von 0,43 g (15,5% d. Theorie) in Form von leicht braunen Öl erhalten (Gehalt nach GC 94%).
MS: **240;** 210 181; 157.

### Beispiel 4 (Eintopfverfahren)

### Herstellung von 2-(4,6-Dimethoxypyrimidin-2-yl-oxy)-3-methylbenzoesäuremethylester

4,38 g (25 mmol) 2-Chlor-4,6-dimethoxypyrimidin, 4,17 g (25,0 mmol) 2-Hydroxy-3-methylbenzoesäuremethylester und 0,66 g (6,3 mmol) Natriummethansulfinat wurden in Gegenwart von 5,17 g (37,5 mmol) Kaliumcarbonat in 25 ml N,N-Dimethylformamid unter Rühren auf 120 °C erhitzt. Nach 8 h wurde das Lösungsmittel bei 60 °C / 20 mbar am Rotavapor entfernt. Der Rückstand wurde aufgenommen in 30 ml Wasser und 30 ml Dichlormethan. Nach Abtrennen der organischen Phase wurde die wässrige Phase erneut mit 20 ml Dichlormethan extrahiert. Die vereinigten organischen Phasen wurden mit Wasser gewaschen, getrocknet über Magnesiumsulfat und eingedampft. Der Rückstand wurde über eine Silicagel-Säule (Eluationsmittel Hexan/ Essigsäureethylester 4:1) chromatographisch gereinigt. Aus der Produktfraktion wurde das Titelprodukt in einer Ausbeute von 5,23 g (65,8% d. Theorie) erhalten (Gehalt nach GC 96%).
Fp. 73,8°C-79,1 °C
   - ¹H NMR (DMSO, MHz 400) δ: 7,75 (1 H, d);
   7,58 (1 H, d);
   7,30 (1 H, t);
   5,95 (1 H, s);
   3,75 (6 H, s);
   3,62 (3 H, s);
   2,17 (3 H, s).
GC/MS: **304**; 273, 245

### Beispiel 5 (Eintopfverfahren)

### Herstellung von 2-(4,6-Dimethoxypyrimidin-2-yl-oxy)-benzoesäuremethylester

4,38 g (25 mmol) 2-Chlor-4,6-dimethoxypyrimidin, 3,80 g (25,0 mmol) 2-Hydroxybenzoesäuremethylester und 0,66 g (6,3 mmol) Natriummethansulfinat wurden in Gegenwart von 5,17 g (37,5 mmol) Kaliumcarbonat in 25 ml N,N-Dimethylformamid unter Rühren auf 120 °C erhitzt. Nach 1,5 h wurde das Lösungsmittel bei 60 °C / 20 mbar am Rotavapor entfernt. Der Rückstand wurde aufgenommen in 30 ml Wasser und 30 ml Dichlormethan. Nach Abtrennen der organischen Phase wurde die wässrige Phase erneut mit 20 ml Dichlormethan extrahiert.
Die vereinigten organischen Phasen wurden mit Wasser gewaschen, getrocknet über Magnesiumsulfat und eingedampft. Der Rückstand wurde über eine Silicagel-Säule (Eluationsmittel Hexan/ Essigsäureethylester 4:1) chromatographisch gereinigt. Aus der Produktfraktion wurde das Titelprodukt in einer Ausbeute von 5,76 g (77,0% d. Theorie) erhalten (Gehalt nach GC 97%).
Fp. 106,7 °C - 108,3 °C
   - ¹H NMR (DMSO, MHz 400) δ: 7,92 (1 H, d);
   7,70 (1 H, t);
   7,40 (1 H, t);
   7,32 (1 H, d);
   5,95 (1 H, s);
   3,75 (6 H, s);
   3,62 (3 H, s).
GC / MS: **290**; 231.

### Beispiel 6 (Eintopfverfahren)

### Herstellung von 3-(4,6-Dimethoxypyrimidin-2-yl-oxy)-2-pyridincarbonsäuremethylester

1,75 g (10 mmol) 2-Chlor-4,6-dimethoxypyrimidin, 1,53 g (10 mmol) 3-Hydroxy-2-pyridincarbonsäuremethylester und 0,104 g (1,0 mmol) Natriummethansulfinat wurden in Gegenwart von 2,17 g (15 mmol) Kaliumcarbonat in 6 ml N,N-Dimethylformamid unter Rühren auf 100 °C erhitzt. Nach 5 h wurde das Lösungsmittel bei 60 °C / 20 mbar am Rotavapor entfernt. Der Rückstand wurde über eine Silicagel-Säule (Eluationsmittel Hexan/Essigsäureethylester 4:1) chromatographisch gereinigt. Aus der Produktfraktion wurde das Titelprodukt in Form eines gelblichen Öls in einer Ausbeute von 1,94 g (61% d. Theorie) erhalten (Gehalt nach GC 92,3%).
- ¹H NMR (DMSO, MHz 400) δ: 8,58 (1 H, d);
7,90 (1 H, d);
7.74 (1 H, dd);
6,01 (1 H, s);
3,75 (6 H, s); 3,68 (3 H, s).

## Patentansprüche

1. Verfahren zur Herstellung von substituierten Pyrimidinderivaten der allgemeinen Formel 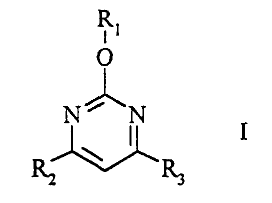 worin R₁ die Bedeutung hat von
i) 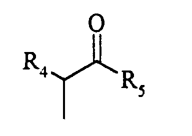 worin R₄ die Bedeutung hat von H, gegebenenfalls mit (C₁-C₄)-Alkyl, Halogen, (C₁-C₄)-Haloalkyl, (C₁-C₄)-Alkoxy, (C₁-C₄)-Haloalkoxy, Nitro oder Cyano substituiertem (C₁-C₆)-Alkyl, (C₁-C₆)-Alkylcarbonyl, (C₁-C₆)-Alkoxycarbonyl, Phenyl, Phenyl-(C₁-C₆)-alkyl, Naphthyl, Naphthyl-(C₁-C₆)-alkyl oder von Carbonyl und worin R₅ die Bedeutung hat von Hydroxy, gegebenenfalls wie oben substituiertem (C₁-C₆)-Alkyl, (C₁-C₆)-Alkoxy, Phenyloxy oder Naphthyloxy
ii) 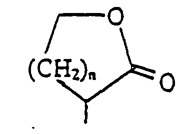 worin n die Bedeutung hat von 1 oder 2
iii) 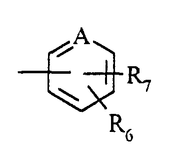 worin A die Bedeutung hat von CH oder von N und worin R₆ und R₇ gleich oder verschieden sind und Wasserstoff oder gegebenenfalls wie oben substituiertes (C₁-C₆)-Alkyl, (C₁-C₆)-Alkoxy, Phenyl, Naphthyl, Phenyloxy oder Naphthyloxy oder eine Gruppe
-COOR₈
bedeuten, worin R₈ die Bedeutung hat von Wasserstoff oder gegebenenfalls wie oben substituiertem (C₁-C₆)-Alkyl, (C₁-C₆)-Alkoxy, Phenyl oder Naphthyl,
R₂ und R₃ gleich oder verschieden sind und H oder gegebenenfalls wie oben substituiertes (C₁-C₆)-Alkyl, (C₁-C₆)-Alkoxy, (C₁-C₆)-Alkylthio, Halogen, Halo-(C₁-C₆)-alkoxy, Amino, (C₁-C₆)-Alkylamino oder Di-(C₁-C₆)-alkylamino bedeuten,
**dadurch gekennzeichnet, dass** ein Halogenpyrimidin der allgemeinen Formel 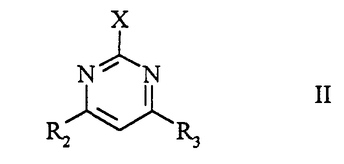 worin X ein Halogenatom bedeutet und R₂ und R₃ die genannte Bedeutung haben, mit einer Hydroxyverbindung, ausgewählt aus der Reihe 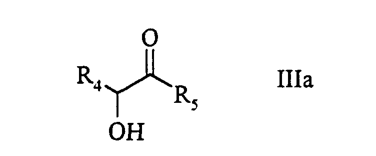 worin R₄ und R₅ die genannte Bedeutung haben 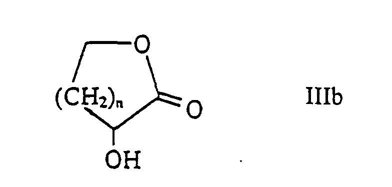 worin n die genannte Bedeutung hat 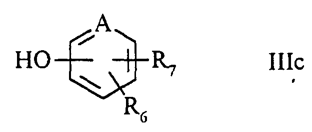 worin A, R₆ und R₇ die genannte Bedeutung haben, in Gegenwart von einem Sulfinat der allgemeinen Formel
R₉SO₂⁻M⁺ IV
worin R₉ H, (C₁-C₆)-Alkyl, gegebenen falls mit (C₁-C₄)-Alkyl substituiertes Phenyl, Phenyl-(C₁-C₆)-alkyl, Naphthyl, Naphthyl-(C₁-C₆)-alkyl, (C₁-C₆)-Alkoxyphenyl oder (C₁-C₆)-Alkoxynaphthyl bedeutet und M ein Alkali- oder Erdalkalimetallatom bedeutet, und in Gegenwart einer anorganischen oder organischen Base zum Endprodukt umgesetzt wird.

2. Verfahren nach Anspruch 1, **dadurch gekennzeichnet, dass** die Umsetzung in Gegenwart von katalytischen Mengen des Sulfinats der allgemeinen Formel IV erfolgt.

3. Verfahren nach Anspruch 1 oder 2, **dadurch gekennzeichnet, dass** das Sulfinat der allgemeinen Formel IV in einer Menge von 1 bis 25 Mol%, bezogen auf das eingesetzte Halogenpyrimidin der allgemeinen Formel II, eingesetzt wird.

4. Verfahren nach einem der Ansprüche 1 bis 3, **dadurch gekennzeichnet, dass** im Sulfinat der allgemeinen Formel IV M⁺ ein Alkalimetallatom, ausgewählt aus der Reihe Natrium, Kalium und Lithium, bedeutet und R₉ (C₁-C₄)-Alkyl, Phenyl-(C₁-C₄)-alkyl oder gegebenenfalls mit (C₁-C₄)-Alkyl substituiertes Phenyl bedeutet.

5. Verfahren nach einem der Ansprüche 1 bis 4, **dadurch gekennzeichnet, dass** eine anorganische Base verwendet wird.

6. Verfahren nach Anspruch 5, **dadurch gekennzeichnet, dass** als anorganische Base ein Alkali- oder ein Erdalkalicarbonat verwendet wird.

7. Verfahren nach einem der Ansprüche 1 bis 6, **dadurch gekennzeichnet, dass** die Umsetzung in Gegenwart eines polaren Lösungsmittels durchgeführt wird.

8. Verfahren nach einem der Ansprüche 1 bis 7, **dadurch gekennzeichnet, dass** die Umsetzungstemperatur zwischen 50 °C und 150 °C gewählt wird.

## Claims

1. Process for the production of substituted pyrimidine derivatives of the general formula 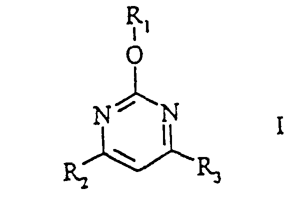 in which R₁ has the meaning of
i) 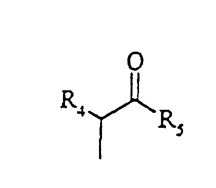 in which R₄ has the meaning of H, of (C₁-C₆)-alkyl, (C₁-C₆) -alkylcarbonyl, (C₁-C₆)-alkoxycarbonyl, phenyl, phenyl-(C₁-C₆)-alkyl, naphthyl, naphthyl- (C₁-C₆) -alkyl, optionally substituted with (C₁-C₄)-alkyl, halogen, (C₁-C₄) -haloalkyl, (C₁-C₄)-alkoxy, (C₁-C₄) -haloalkoxy, nitro or cyano, or of carbonyl and in which R₅ has the meaning of hydroxyl, of (C₁-C₆)-alkyl, (C₁-C₆)-alkoxy, phenyloxy or naphthyloxy, optionally substituted as above,
ii) 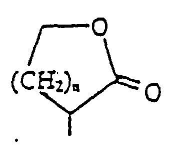 in which n has the meaning of 1 or 2
iii) 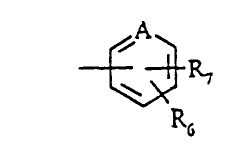 in which A has the meaning of CH or of N and in which R₆ and R₇ are identical or different and mean hydrogen or (C₁-C₆)-alkyl, (C₁-C₆)-alkoxy, phenyl, naphthyl, phenyloxy or naphthyloxy, optionally substituted as above, or a group
-COOR₈
in which R₈ has the meaning of hydrogen or of (C₁-C₆)-alkyl, (C₁-C₆)-alkoxy, phenyl or naphthyl, optionally substituted as above,
R₂ and R₃ are identical or different and mean H or (C₁-C₆)-alkyl, (C₁-C₆) -alkoxy, (C₁-C₆)-alkylthio, halogen, halo- (C₁-C₆) -alkoxy, amino, (C₁-C₆)-alkylamino or di-(C₁-C₆)-alkylamino, optionally substituted as above,
**characterised in that** a halopyrimidine of the general formula 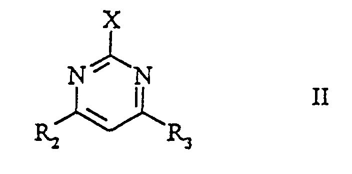 in which X means a halogen atom and R₂ and R₃ have the stated meaning, is reacted with a hydroxy compound selected from the range 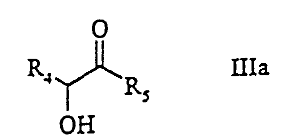 in which R₄ and R₅ have the stated meaning 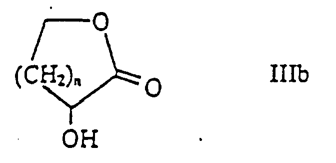 in which n has the stated meaning 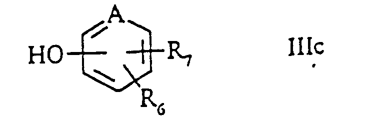 in which A, R₆ and R₇ have the stated meaning, in the presence of a sulfinate of the general formula
R₉SO₂⁻M⁺ IV
in which R₉ means H, (C₁-C₆)-alkyl, or phenyl, phenyl-(C₁-C₆)-alkyl, naphthyl, naphthyl- (C₁-C₆) -alkyl, (C₁-C₆)-alkoxyphenyl or (C₁-C₆)-alkoxynaphthyl, optionally substituted with (C₁-C₄)-alkyl, and M means an alkali metal or alkaline earth metal atom, and in the presence of an inorganic or organic base to yield the final product.

2. Process according to claim 1, **characterised in that** the reaction is performed in the presence of catalytic quantities of the sulfinate of the general formula IV.

3. Process according to claim 1 or 2, **characterised in that** the sulfinate of the general formula IV is used in a quantity of 1 to 25 mol%, relative to the halopyrimidine of the formula II which is used.

4. Process according to one of claims 1 to 3, **characterised in that**, in the sulfinate of the general formula IV, M⁺ means an alkali metal atom, selected from the range sodium, potassium and lithium, and R₉ means (C₁-C₄) -alkyl, phenyl- (C₁-C₄) -alkyl or phenyl optionally substituted with (C₁-C₄)-alkyl.

5. Process according to one of claims 1 to 4, **characterised in that** an inorganic base is used.

6. Process according to claim 5, **characterised in that** an alkali metal or alkaline earth metal carbonate is used as the inorganic base.

7. Process according to one of claims 1 to 6, **characterised in that** the reaction is performed in the presence of a polar solvent.

8. Process according to one of claims 1 to 7, **characterised in that** the reaction temperature is selected between 50°C and 150°C.

## Revendications

1. Procédé de préparation de dérivés de pyrimidines substituées de formule générale 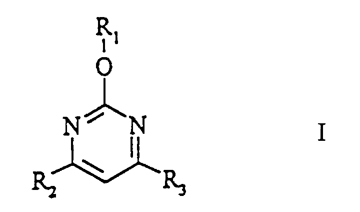 dans laquelle R₁ représente
i) un groupe 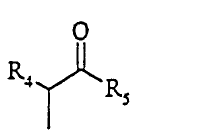 dans lequel R₄ représente H, un groupe alkyle en C₁-C₆, (alkyl en C₁-C₆)carbonyle, (alcoxy en C₁-C₆)carbonyle, phényle, phényl(alkyle en C₁-C₆), naphtyle ou naphtyl(alkyle en C₁-C₆), éventuellement substitué par alkyle en C₁-C₄, halogène, halogénoalkyle en C₁-C₄, alcoxy en C₁-C₄, halogénoalcoxy en C₁-C₄, nitro ou cyano, ou un groupe carboxyle, et R₅ représente un groupe hydroxy ou un groupe alkyle en C₁-C₆, alcoxy en C₁-C₆, phényloxy ou naphtyloxy, éventuellement substitué de la manière décrite ci-dessus;
ii) un groupe 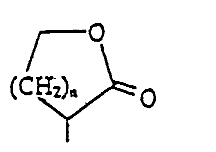 dans lequel n est égal à 1 ou 2;
iii) un groupe 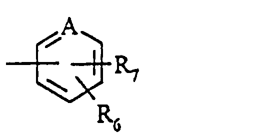 dans lequel A représente CH ou N et R₆ et R₇ sont identiques ou différents et représentent un atome d'hydrogène, un groupe alkyle en C₁-C₆, alcoxy en C₁-C₆, phényle, naphtyle, phényloxy ou naphtyloxy, éventuellement substitué de la manière décrite ci-dessus, ou un groupe -COOR₈, dans lequel R₈ représente un atome d'hydrogène ou un groupe alkyle en C₁-C₆, alcoxy en C₁-C₆, phényle ou naphtyle, éventuellement substitué de la manière décrite ci-dessus, R₂ et R₃ sont identiques ou différents et représentent H ou un groupe alkyle en C₁-C₆, alcoxy en C₁-C₆, alkylthio en C₁-C₆, halogéno, halogénoalcoxy en C₁-C₆, amino, alkylamino en C₁-C₆ ou di(alkyl en C₁-C₆)amino, éventuellement substitué de la manière décrite ci-dessus,
**caractérisé en ce que** l'on fait réagir une halogénopyrimidine de formule générale 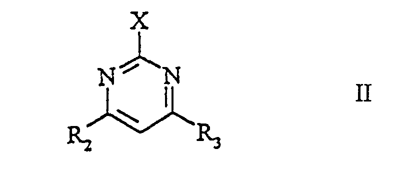 dans laquelle X représente un atome d'halogène et R₂ et R₃ ont la signification indiquée, avec un composé hydroxy choisi dans la série de 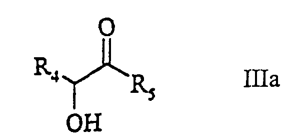 où R₄ et R₅ ont la signification indiquée, 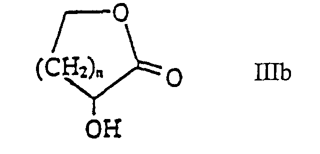 où n a la signification indiquée, et 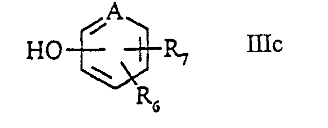 où A, R₆ et R₇ ont la signification indiquée,
en présence d'un sulfinate de formule générale
R₉SO₂⁻M⁺ IV
dans laquelle R₉ représente H ou un groupe alkyle en C₁-C₆, phényle éventuellement substitué par alkyle en C₁-C₄, phényl(alkyle en C₁-C₆), naphtyle, naphtyl(alkyle en C₁-C₆), (alcoxy en C₁-C₆)phényle ou (alcoxy en C₁-C₆)naphtyle, et M est un atome de métal alcalin ou de métal alcalino-terreux, et en présence d'une base inorganique ou organique, pour obtenir le produit final.

2. Procédé selon la revendication 1, **caractérisé en ce que** la réaction s'effectue en présence de quantités catalytiques du sulfinate de formule générale IV.

3. Procédé selon la revendication 1 ou 2, **caractérisé en ce que** l'on utilise le sulfinate de formule générale IV en une quantité de 1 à 25 % en mol par rapport à l'halogénopyrimidine de formule générale II utilisée.

4. Procédé selon l'une des revendications 1 à 3, **caractérisé en ce que**, dans le sulfinate de formule générale IV, M⁺ représente un atome de métal alcalin choisi dans la série du sodium, du potassium et du lithium, et R₉ représente un groupe alkyle en C₁-C₄, phényl(alkyle en C₁-C₄) ou phényle éventuellement substitué par alkyle en C₁-C₄.

5. Procédé selon l'une des revendications 1 à 4, **caractérisé en ce que** l'on utilise une base inorganique.

6. Procédé selon la revendication 5, **caractérisé en ce que** l'on utilise comme base inorganique un carbonate de métal alcalin ou de métal alcalino-terreux.

7. Procédé selon l'une des revendications 1 à 6, **caractérisé en ce que** la réaction s'effectue en présence d'un solvant polaire.

8. Procédé selon l'une des revendications 1 à 7, **caractérisé en ce que** l'on choisit la température de la réaction entre 50°C et 150°C.
